# FASCICULE DE BREVET EUROPEEN

(11) **EP 2 380 117 B1**
(45) Date de publication et mention de la délivrance du brevet: **12.08.2020**
(21) Numéro de dépôt: 09805792.0
(22) Date de dépôt: 28.12.2009
(51) Int. Cl.: G06M 1/16, G06M 1/24

(54) **COMPTEUR POUR DISPOSITIF DE DISTRIBUTION DE PRODUIT FLUIDE OU PULVERULENT**
ZÄHLEINRICHTUNG FÜR FLÜSSIGKEITS- ODER PULVERABGABEVORRICHTUNG
COUNTER FOR FLUID OR POWDER DISPENSER

(30) Priorité: 30.12.2008 FR 0859142
(43) Date de publication de la demande: 26.10.2011
(73) Titulaire: Aptar France SAS, 27110 Le Neubourg (FR)
(72) Inventeur: LAUT, Antoine, 27150 Etrepagny (FR)
(74) Mandataire: CAPRI
(86) Numéro de dépôt international: PCT/FR2009/052705
(87) Numéro de publication internationale: WO 2010/076528

(56) Documents cités:
- WO-A-2007/077450
- WO-A-2007/104964
- WO-A2-99/36115

## Description

La présente invention concerne un compteur de doses pour indiquer à l'utilisateur le nombre de doses distribuées ou restant à distribuer à partir d'un dispositif de distribution de produit fluide ou pulvérulent.

L'utilisation de compteurs ou d'indicateurs est bien connue dans le domaine des distributeurs de produit fluide, notamment dans le domaine pharmaceutique. Ces compteurs ou indicateurs sont notamment utilisés avec des dispositifs de distribution du type MDI (Metered Dose Inhalers), dans lesquels un réservoir contenant du produit fluide et un gaz propulseur est monté déplaçable dans un corps, le déplacement dudit réservoir provoquant l'actionnement d'une valve doseuse montée sur ledit réservoir pour distribuer une dose de produit. Une première famille de compteurs prévoit de fixer le compteur sur le fond du réservoir faisant saillie hors du corps, et sur lequel l'utilisateur appuie pour distribuer une dose. Ce type de compteur présente toutefois l'inconvénient d'interférer avec l'actionnement du dispositif de distribution, l'utilisateur devant appuyer sur le compteur pour actionner le dispositif. En cas d'actionnement mal maîtrisé ou partiel, il peut alors se poser des problèmes de sur- et/ou sous-comptage et/ou de distribution incomplète ou défaillante. Une seconde famille de compteurs comprend des compteurs disposés à l'intérieur du corps en étant fixés soit au corps, soit au réservoir mobile dans ledit corps. Ce type de compteur présente notamment l'inconvénient d'un montage complexe et nécessite des modifications substantielles des différentes parties constitutives du dispositif de distribution. Le problème de l'assemblage se pose notamment lorsque cet assemblage est réalisé chez le fabricant du produit pharmaceutique et non chez le fabricant du dispositif de distribution, obligeant le fabricant du produit à installer des machines d'assemblages complexes dans son usine. Une troisième famille de compteurs prévoit de disposer le compteur latéralement à l'extérieur du corps, une projection dudit compteur passant à travers une ouverture du corps pour coopérer avec le réservoir ou une partie solidaire de celui-ci. Ce type de compteurs nécessite généralement aussi une modification substantielle du corps afin de recevoir le compteur. Par ailleurs, la présence d'un compteur sur la face latérale externe du corps modifie sensiblement l'aspect extérieur du dispositif, en particulier en raison de l'épaisseur dudit compteur, ce qui peut aussi avoir un effet négatif sur la maniabilité du dispositif. Par ailleurs, plusieurs contraintes reposent sur des compteurs utilisés avec des dispositifs de distribution de produits, notamment pharmaceutiques. Ainsi, pour éviter tout risque de sous-comptage, il est généralement exigé que l'actionnement du compteur soit réalisé au tout début de la course d'actionnement de la valve ou de la pompe, pour éviter qu'un actionnement partiel ne distribue une dose partielle ou complète, mais qui ne serait pas comptée par le compteur. Un problème qui se pose dans ce cas est que cette course d'actionnement est généralement très courte et que les tolérances du dispositif ont tendance à encore d'avantage réduire la distance disponible de manière effective pour réaliser cet actionnement. Ceci nécessite généralement l'utilisation d'un mécanisme complexe pour fournir un comptage fonctionnel et sûr. De manière générale, l'assemblage des compteurs, notamment ceux comportant plusieurs éléments rotatifs imbriqués les uns dans les autres, s'avère complexe et donc non seulement coûteuse mais aussi sources de dysfonctionnements. Les documents WO 2007/077450, WO 99/36115 A2 et WO 2007/104964 décrivent des compteurs de l'art antérieur.

La présente invention a pour but de fournir un compteur, plus particulièrement un compteur de doses pour dispositif de distribution de produit fluide ou pulvérulent, qui ne reproduit pas les inconvénients susmentionnés.

La présente invention a notamment pour but de fournir un tel compteur qui présente une épaisseur minimale.

La présente invention a aussi pour but de fournir un tel compteur qui puisse être pré-assemblé avant livraison au fabricant du produit pharmaceutique, celui-ci n'ayant plus alors qu'à réaliser une simple étape de montage du compteur sur le corps du dispositif de distribution, sans assemblage complexe des parties constitutives dudit compteur.

La présente invention a aussi pour but de fournir un tel compteur qui garantit un actionnement du compteur indépendamment de la longueur de la course d'actionnement de la pompe ou de la valve utilisée dans le dispositif.

La présente invention a encore pour but de fournir un tel compteur qui soit plus simple et donc moins coûteux à fabriquer et à assembler, et plus fiable de fonctionnement.

La présente invention a donc pour objet un dispositif selon la revendication 1. Des modes de réalisation avantageux sont décrits dans les revendications dépendantes.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
- la figure 1 est une vue éclatée en perspective d'un dispositif de distribution comportant sur sa face latérale avant compteur selon un mode de réalisation particulier de la présente invention ;
- les figures 2a et 2b sont deux vues schématiques en perspectives du compteur pré-assemblé de la figure 1, respectivement vue de devant et de derrière ;
- la figure 3 est une vue schématique en perspective de l'organe d'actionnement selon un mode de réalisation avantageux de l'invention ;
- la figure 4 est une vue schématique en perspective partiellement transparente des deux éléments de comptage rotatifs et de l'élément intermédiaire rotatif, selon un mode de réalisation avantageux de l'invention, en position d'assemblage de l'élément intermédiaire ;
- la figure 5 est une vue schématique découpée en perspective du compteur selon un mode de réalisation avantageux,
- la figure 6 est une vue schématique en coupe transversale du compteur de la figure 5,
- les figures 7a et 7b représentent des vues schématiques respectivement de devant et de derrière du premier élément de comptage rotatif, selon une variante de réalisation avantageuse,
- les figures 8a et 8b représentent des vues schématiques respectivement de devant et de derrière du second élément de comptage rotatif, selon une variante de réalisation avantageuse,
- les figures 9a et 9b représentent des vues schématiques respectivement de devant et de derrière de l'élément intermédiaire rotatif, selon une variante de réalisation avantageuse,
- la figure 10 est une vue schématique en perspective de dessus d'un compteur selon un mode de réalisation avantageux de la présente invention ;
- la figure 11 est une vue similaire à celle de la figure 10, mais vue de dessous ;
- la figure 12 est une vue schématique en perspective partiellement transparente des deux éléments de comptage rotatifs et de l'élément intermédiaire rotatif, selon un mode de réalisation avantageux de l'invention, en position d'assemblage de l'élément intermédiaire ;
- la figure 13 est une vue schématique en coupe transversale du compteur de la figure 12 ;
- la figure 14 est une vue schématique découpée en perspective et partiellement transparente de l'organe d'actionnement du compteur selon un mode de réalisation avantageux, assemblé dans le corps de base ;
- la figure 15 est une vue similaire à celle de la figure 14, mais vue de dessous ;
- les figures 16a et 16b représentent des vues schématiques respectivement de devant et de derrière du second élément de comptage rotatif, selon une variante de réalisation avantageuse ; et
- les figures 17a et 17b représentent des vues schématiques respectivement de devant et de derrière du premier élément de comptage rotatif, selon une variante de réalisation avantageuse.

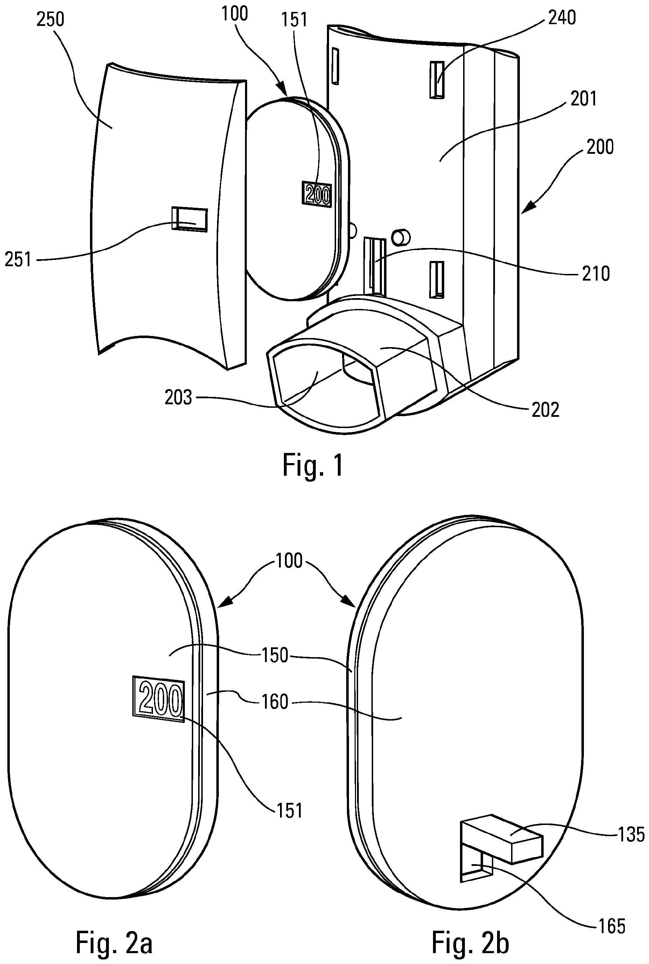

En référence à la figure 1, il est représenté un dispositif de distribution du type MDI 200, comportant sur sa face latérale avant un compteur de doses 100 qui correspond à un mode de réalisation particulier de la présente invention. Le dispositif comporte un corps 201 pourvu d'un embout buccal 202 définissant un orifice de distribution 203. Un réservoir (non représenté) est assemblé dans ledit corps 201, ledit réservoir comportant une valve doseuse monté sur son ouverture. Cette valve doseuse coopère à l'intérieur dudit corps 201 avec un canal d'expulsion menant dans l'embout buccal 202. Lorsque l'utilisateur appuie sur le fond du réservoir, il fait coulisser celui-ci axialement à l'intérieur du corps 201, provoquant l'actionnement de la valve et l'expulsion d'une dose de produit fluide. Le fonctionnement d'un tel dispositif de type MDI est bien connu de l'homme du métier, et il ne sera donc pas plus amplement décrit ici. Le compteur 100 peut être intercalé entre la face latérale avant dudit corps 201 et un organe de recouvrement 250 comportant une fenêtre 251, et qui peut servir à fixer le compteur 100 sur le corps. Bien entendu, cette mise en œuvre n'est qu'un exemple de réalisation, et par exemple, le compteur pourrait directement être fixé sur le corps 201, indépendamment de la présence d'un organe de recouvrement. Le compteur comporte un élément d'actionnement 135 qui se projette hors dudit compteur, et qui est adapté à pénétrer à l'intérieur du corps 201 à travers une ouverture 210 prévue à cet effet, pour coopérer avec le réservoir ou une partie solidaire de celui-ci. De cette manière, à chaque actionnement du dispositif de distribution, le déplacement axial du réservoir dans le corps 201 provoque le déplacement axial de l'élément d'actionnement 135.

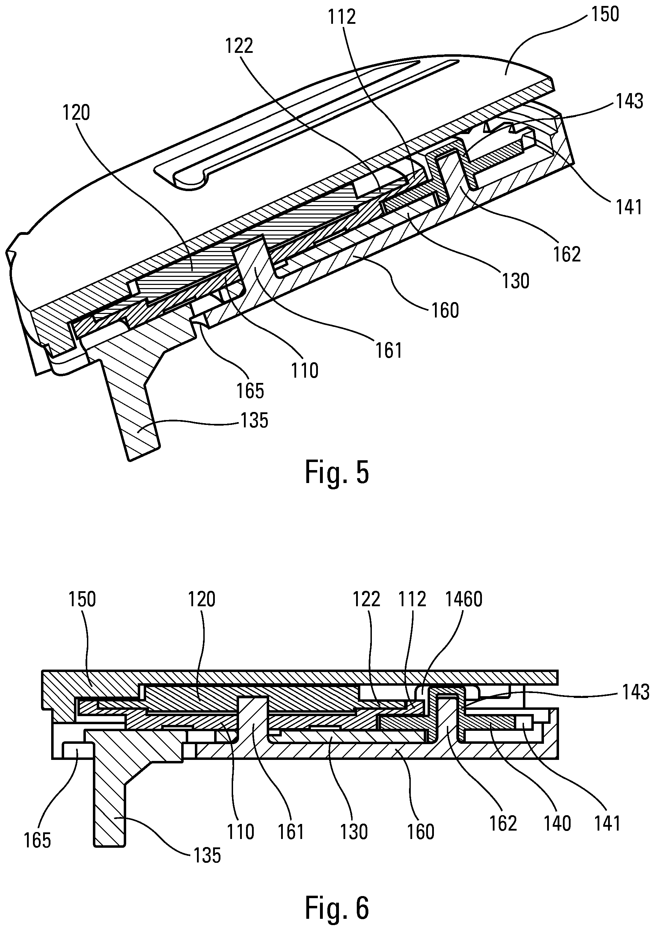

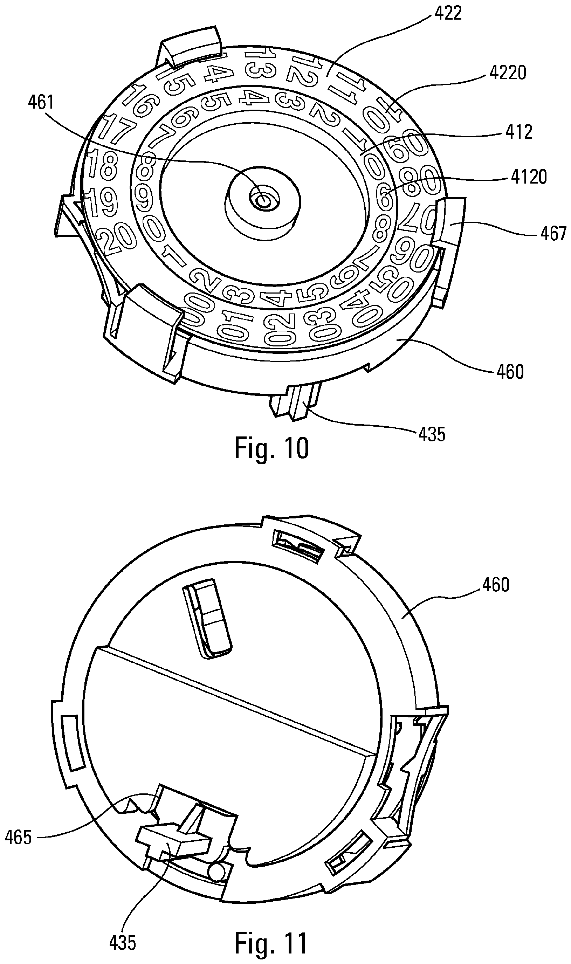

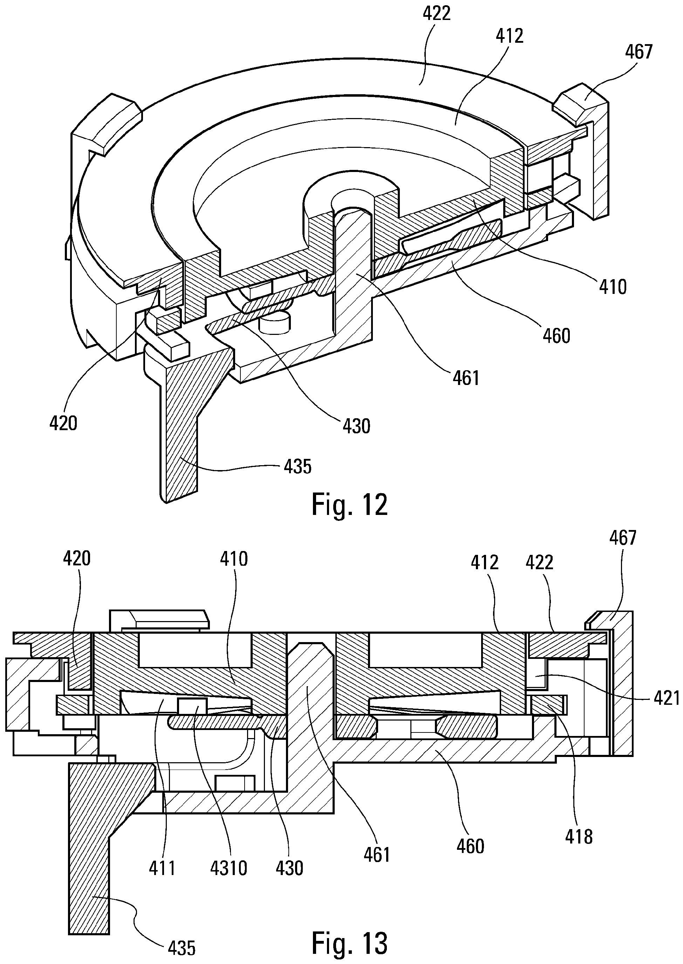

Les figures 1 à 9 représentent un premier mode de réalisation avec deux éléments de comptage rotatifs et un élément intermédiaire également rotatif, et les figures 10 à 17 représentent un second mode de réalisation, avec deux éléments de comptage rotatifs mais sans élément intermédiaire.

Le compteur selon le premier mode de réalisation de l'invention comporte deux éléments de comptage rotatifs, à savoir un premier élément de comptage rotatif 110 et un second élément de comptage rotatif 120, un organe d'actionnement 130 et un élément intermédiaire rotatif 140. L'organe d'actionnement 130, qui comporte l'élément d'actionnement 135, est prévu pour transformer un déplacement axial d'une partie du dispositif de distribution 200, en général le réservoir, en un déplacement rotatif du premier élément de comptage 110.

Comme représenté sur la figure 1, le compteur est disposé latéralement par rapport au corps 201 du dispositif de distribution 200, et l'organe d'actionnement 130 transforme alors un déplacement axial du réservoir en un déplacement rotatif du premier élément de comptage 110. Dans ce cas, les trois éléments rotatifs 110, 120, 140 du compteur tournent autour d'axes de rotation 161, 162 sensiblement perpendiculaires à ce déplacement axial. Avantageusement, le cycle d'actionnement du compteur peut démarrer au tout début de la course du réservoir, de sorte que le compteur est actionné avant que le produit ne soit distribué.

Le premier élément de comptage rotatif 110 forme la roue des unités et le second élément de comptage rotatif 120 forme la roue des dizaines, lesdits premier et second éléments de comptage coopérant pour définir ensemble et afficher dans une fenêtre de visualisation 151 le nombre de doses de produit fluide distribué ou restant à distribuer dudit réservoir. De préférence, ce nombre est formé par une zone d'affichage dans laquelle le nombre est affiché horizontalement lorsque le dispositif de distribution 200 est dans sa position d'utilisation normale, représentée sur la figure 1, dans laquelle le corps 201 est sensiblement vertical avec l'embout buccal 202 disposé en bas. Ledit premier élément de comptage 110 coopère avec l'organe d'actionnement 130 qui est adapté à faire tourner ledit premier élément de comptage 110 à chaque actionnement dudit organe d'actionnement. L'élément intermédiaire rotatif 140 est adapté à faire tourner ledit second élément de comptage 120 tous les dix actionnements dudit organe d'actionnement 130, et donc toutes les dix rotations dudit premier élément de comptage 110. Lesdits premier et second éléments de comptage 110, 120 tournent autour d'un même premier axe de rotation 161, et ledit élément intermédiaire 140 tourne autour d'un second axe de rotation 162, décalé et parallèle audit premier axe de rotation 161.

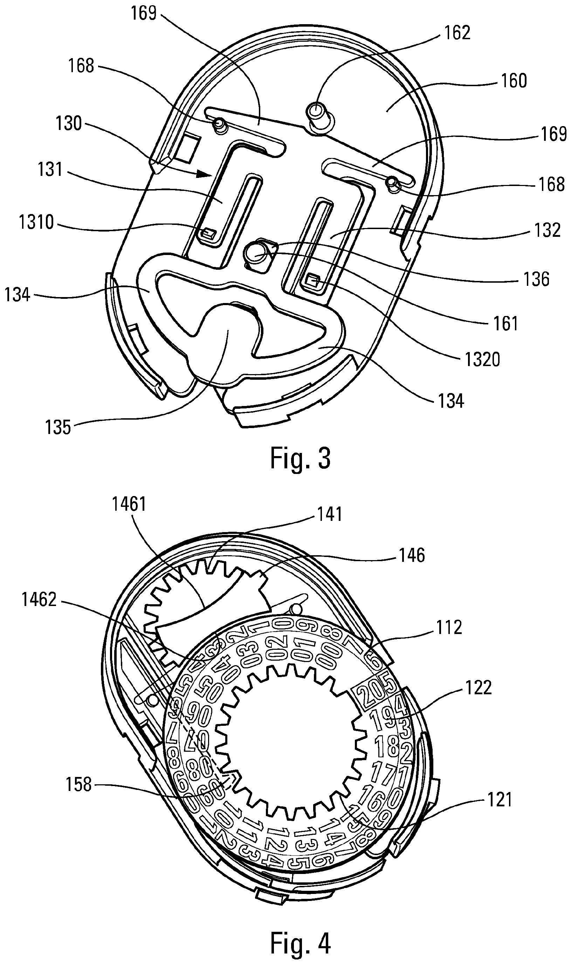

Comme représenté sur les figures 3, 5 et 6, le compteur comporte de préférence un corps de base 160 et un couvercle 150. Ledit corps de base forme les deux axes de rotation 161 et 162 et comporte une ouverture 165 destinée à laisser passer l'élément d'actionnement 135. Le couvercle 150 comporte une fenêtre de visualisation 151 permettant à l'utilisateur de voir la zone d'affichage formée conjointement par lesdits premier et second éléments de comptage. Ainsi, ledit compteur peut avantageusement être pré-assemblé pour former une unité de compteur, ladite unité de compteur pouvant comporter des moyens de fixation au corps 201 du dispositif de distribution de produit fluide 200. Ces moyens de fixation sont formés sur ledit corps de base.

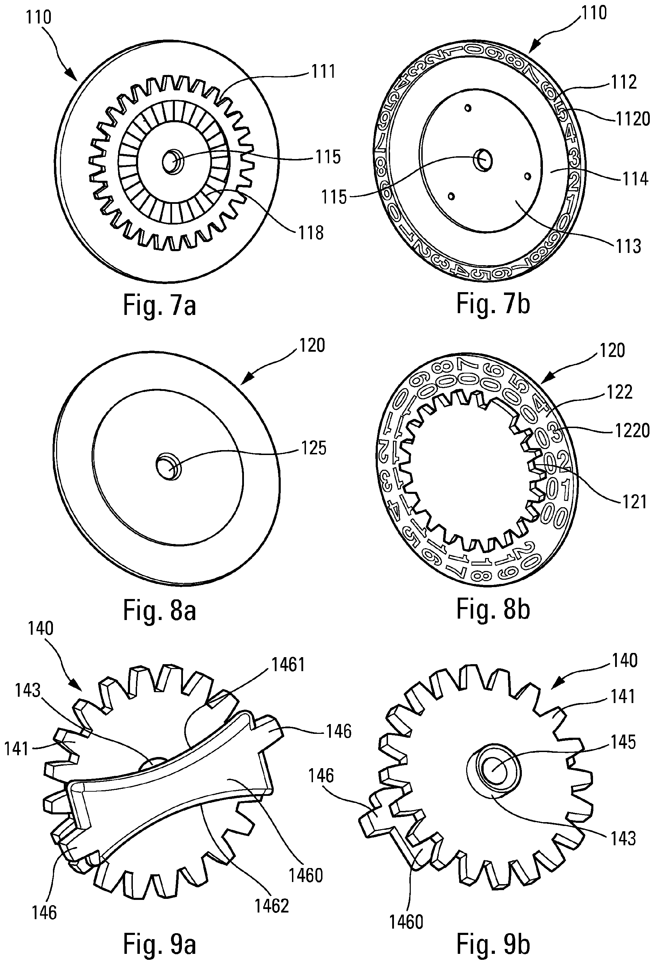

Comme représenté sur les figures 7a et 7b, ledit premier élément de comptage 110 peut comporter une première denture périphérique 118 coopérant à chaque actionnement avec au moins une, de préférence deux pattes d'actionnement flexibles 131, 132 de l'organe d'actionnement 130. Une seconde denture périphérique 111 est prévue pour coopérer à chaque actionnement avec l'élément intermédiaire 140. Ledit premier élément de comptage 110 est avantageusement sensiblement en forme de disque en étant pourvu d'une ouverture centrale traversante 115 adaptée à s'emmancher autour de son axe de rotation 161. La face supérieure dudit disque comporte une première partie de bord périphérique radialement externe 112 recevant des indices de comptage 1120, tel qu'une ou plusieurs séries de chiffres de 0 à 9. L'exemple représenté sur la figure 7b montre trois séries de chiffres de 0 à 9, répartis sur ladite périphérie. La face inférieure dudit disque comporte lesdites première et seconde dentures périphériques 118, 111, comme visible sur la figure 7a. De préférence, la première denture 118 est radialement à l'intérieur de la seconde denture 111, les dents de la première denture 118 étant orientées axialement, alors que les dents de la seconde denture sont orientées radialement vers l'extérieur. La première denture 118 et les deux pattes flexibles 131, 132 forment aussi des moyens anti-retour empêchant le premier élément de comptage 110 de tourner en sens inverse à celui qui lui est impartit par l'organe d'actionnement 130. Comme visible sur la figure 7b, la face supérieure dudit premier élément de comptage 110 peut comporter une partie centrale 113 entourant l'ouverture centrale 115, prolongée radialement à l'extérieur par une partie intermédiaire 114 axialement surélevée par rapport à ladite partie centrale 113. Ladite partie intermédiaire 114 est alors prolongée radialement à l'extérieur par ladite première partie de bord périphérique 112 axialement surélevée par rapport à ladite partie intermédiaire. Cette mise en œuvre permet de superposer le second élément de comptage 120 sur le premier élément de comptage avec une épaisseur globale réduite.

Comme représenté sur les figures 8a et 8b, ledit second élément de comptage 120 peut comporter une troisième denture périphérique 121 adaptée à coopérer tous les dix actionnements de l'organe d'actionnement 130 avec ledit élément intermédiaire 140. Avantageusement, ledit second élément de comptage est également sensiblement en forme de disque, en étant pourvu d'un orifice central 125, qui dans l'exemple représenté est borgne, et qui est adapté à s'emmancher autour de son axe de rotation 161. La face supérieure dudit disque comporte une seconde partie de bord périphérique radialement externe 122 recevant des indices de comptage 1220, tel que les chiffres de 00 à 20, répartis sur ladite périphérie. Dans cet exemple, le compteur est donc capable de compter 200 doses. La face supérieure dudit disque comporte également ladite troisième denture périphérique 121, disposée radialement à l'intérieur de ladite seconde partie de bord périphérique externe 112, ladite troisième denture périphérique 121 étant axialement surélevée par rapport à ladite seconde partie de bord périphérique externe 122. Avantageusement, après assemblage des premier et second éléments de comptage 110, 120 autour de leur axe de rotation commun 161, ladite seconde partie de bord périphérique externe 122 dudit second élément de comptage 120 est disposée radialement à l'intérieur et sensiblement en contact de ladite première partie de bord périphérique externe 112 dudit premier élément de comptage 110, les surfaces supérieures desdites première et secondes parties de bord périphérique 112, 122 étant sensiblement alignées ou coplanaires pour former la zone d'affichage qui sera visible à travers la fenêtre de visualisation 151.

Comme visible sur les figures 9a et 9b, ledit élément intermédiaire 140 peut comporter une quatrième denture périphérique 141 coopérant à chaque actionnement de l'organe d'actionnement 130 avec la seconde denture périphérique 111 dudit premier élément de comptage 110. De cette manière, chaque actionnement du dispositif de distribution 200 est transformé par l'organe d'actionnement 130 en une rotation du premier élément de comptage 110. Avantageusement, ledit élément intermédiaire 140 comporte également au moins une projection radiale 146 coopérant tous les dix actionnements de l'organe d'actionnement 130 avec la troisième denture périphérique 121 du second élément de comptage 120. Cette projection radiale 146 peut être formée sur une partie de tige radiale 1460 dudit élément intermédiaire 140. Avantageusement, ledit élément intermédiaire 140 comporte un manchon creux axial central 143, qui dans l'exemple représenté est borgne, et qui définit un orifice central 145 adapté à s'emmancher autour de son axe de rotation 162. La quatrième denture 141 et ladite partie de tige radiale 1460 sont décalées l'une de l'autre le long dudit manchon 143 en définissant entre elles un espace qui peut recevoir la première partie de bord périphérique externe 112 du premier élément de comptage 110 et la seconde partie de bord périphérique externe 122 du second élément de comptage 120. Avantageusement, ladite partie de tige radiale 1460 comporte deux projections 146 radialement externes diamétralement opposées reliées entre elles par une zone de tige cintrée dont les bords latéraux sont de forme sensiblement en arc de cercle inversés, comme clairement visible sur les figures 4 et 9a. Ainsi, dans une orientation appropriée, représentée sur la figure 4, les premier et second éléments de comptage 110, 120 peuvent être assemblés l'un après l'autre sur leur axe de rotation commun 161, après assemblage de l'élément intermédiaire 140 sur son propre axe de rotation 162. La présente invention permet ainsi d'éviter que deux des éléments rotatifs du compteur doivent être assemblées simultanément autour des deux axes de rotation décalés, comme cela est généralement le cas avec des compteurs de ce type.

Avantageusement, ledit organe d'actionnement 130 est assemblé dans ledit corps de base 160. Le couvercle 150 peut comporter une patte flexible 158, visible sur la figure 4, coopérant avec la troisième denture 121 dudit second élément de comptage 120 pour empêcher ledit second élément de comptage 120 de tourner dans un sens ou dans l'autre lorsque l'élément intermédiaire 140 ne coopère pas avec ledit second élément de comptage 120. Bien entendu, ladite patte flexible 158 peut se déformer élastiquement pour permettre une rotation dudit second élément de comptage 120 chaque fois que ledit élément intermédiaire 140 coopère avec ledit second élément de comptage 120, c'est-à-dire tous les dix actionnements dudit organe d'actionnement 130. Avantageusement, des moyens de butée sont prévus pour former une butée au déplacement axial des pattes flexibles 131, 132. Ces moyens de butée peuvent avantageusement être formés par la première projection 161 du corps de base 160 qui peut coopérer avec une fenêtre 136 de l'organe d'actionnement 130. D'autres moyens de butée sont aussi envisageables. Les pattes 131 et 132 supportent chacune un ergot respectif 1310 et 1320 qui coopère avec la première denture 118 du premier élément de comptage 110. Les formes des ergots 1310 et 1320 sont inversées de telle sorte que le premier ergot 1310 pousse une dent de la denture 118 lors de la descente du réservoir dans le corps 201 et que le second ergot 1320 tire une dent lors de la remontée du réservoir dans le corps 201. Avantageusement, les pattes flexibles 131 et 132 sont sensiblement rigides axialement et sont flexibles dans une direction perpendiculaire au déplacement axial de l'organe d'actionnement 130. Ceci permet à la patte élastique qui n'agit pas pour faire tourner le premier élément de comptage 110 de se déformer pour glisser sur la denture et s'engrener dans la prochaine dent. Les deux pattes élastiques 131 et 132 forment aussi les moyens anti-retour pour le premier élément de comptage 110. Avantageusement, l'organe d'actionnement 130 comporte des moyens élastiques 169, tels que deux lames élastiques qui coopèrent avec deux épaulements appropriés 168 du corps de base 160 pour former ressort de rappel pour l'organe d'actionnement 130. De préférence, l'organe d'actionnement 130 comporte en outre une partie axialement déformable 134 supportant l'élément d'actionnement 135. Ceci permet de poursuivre le déplacement axial de l'élément d'actionnement 135 (et donc du réservoir) après que la position de butée définie par la projection 161 et la fenêtre 136 a été atteinte. Cette butée peut être formée de telle sorte qu'exactement une rotation d'une demi-dent soit obtenue lors de la descente du réservoir (lorsque la première dent pousse la denture 118) et que la rotation de la demi-dent restante soit obtenue lors de la remontée du réservoir, et donc de l'organe d'actionnement 130 sous l'effet des moyens élastiques 169 (lorsque la seconde dent tire sur la denture 118). L'actionnement de la valve nécessitant généralement une course supérieure, et donc un déplacement axial supérieur du réservoir, la partie déformable 134 de l'organe d'actionnement 130 permet la poursuite du déplacement axial du réservoir jusqu'à son terme. Par ailleurs, ce système permet d'actionner le compteur avant le début de la distribution du produit.

La présente invention permet notamment de sensiblement simplifier le procédé d'assemblage d'un compteur. Ainsi, l'organe d'actionnement 130 est d'abord assemblé dans ledit corps de base 160, avec l'élément d'actionnement 135 s'étendant hors dudit corps de base 160 à travers ladite ouverture 165. Ensuite, l'élément intermédiaire rotatif 140 est assemblé sur sa projection 162 dudit corps de base. Après orientation appropriée dudit élément intermédiaire dans la position d'assemblage représentée sur la figure 4, le premier élément de comptage rotatif 110 puis le second élément de comptage rotatif 120 peuvent être assemblés sur leur projection 161. Enfin, le couvercle 150 peut être assemblé sur ledit corps de base 160, pour former une unité de compteur pré-assemblée.

Le compteur de l'invention présente aussi l'avantage d'être très peu épais, permettant ainsi de diminuer les dimensions externes du dispositif et de ne pas modifier substantiellement la maniabilité du dispositif. En particulier, l'unité de compteur pré-assemblée, incluant le corps de base 160, l'organe d'actionnement 130, l'élément intermédiaire 140, les premier et second éléments de comptage 110, 120 et le couvercle 150 peut avoir une épaisseur inférieure ou égale à 7 mm, avantageusement inférieure à 6 mm, notamment inférieure à 5 mm. Ces mesures ne tiennent bien entendu pas compte de l'élément d'actionnement 135 qui se projette hors de l'unité de compteur pré-assemblée dans le sens de l'épaisseur.

De manière avantageuse, l'actionnement du compteur se fait en deux parties, une première partie avant distribution du produit fluide à travers l'orifice de distribution 203 du corps 201, et une seconde partie après distribution de produit fluide. Avantageusement, le compteur ne fonctionne pas pendant que le produit est effectivement distribué, et son fonctionnement sure et fiable est donc totalement indépendant de la manière dont l'utilisateur actionne le dispositif pour réaliser une distribution de produit.

Bien entendu, par rapport à la description faite ci-dessus, le compteur pourrait être réalisé d'une manière différente à celle représenté. En particulier, les formes et positions des première et seconde pattes flexibles 131 et 132 pourraient être différentes. On peut aussi envisager d'inverser les fonctions des première et seconde pattes flexibles 131, 132, à savoir que la première patte flexible 131 pourrait tirer le premier élément de comptage 110 alors que la seconde patte flexible 132 pourrait le pousser. De même, la forme de la partie déformable 134 supportant l'élément d'actionnement 135 pourrait être différente de celle représentée sur la figure 3.

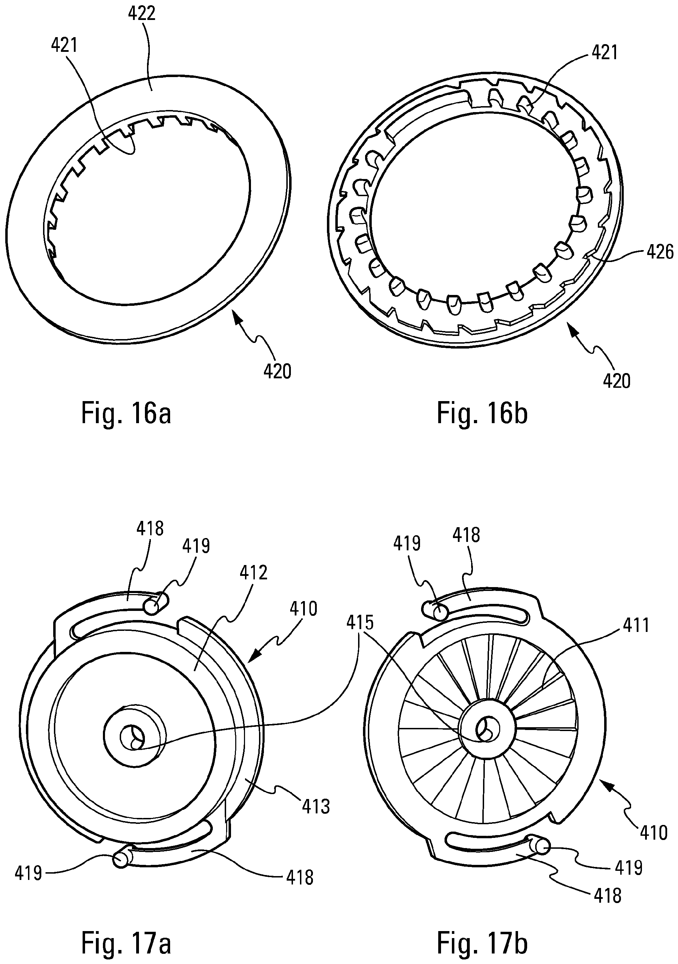

Le compteur selon le deuxième mode de réalisation de l'invention comporte aussi deux éléments de comptage rotatifs, à savoir un premier élément de comptage rotatif 410, représenté en détails sur les figures 17a et 17b, et un second élément de comptage rotatif 420, représenté en détails sur les figures 16a et 16b, ainsi qu'un organe d'actionnement 430. L'organe d'actionnement 430, qui comporte l'élément d'actionnement 435, est prévu pour transformer un déplacement axial d'une partie du dispositif de distribution, en général le réservoir, en un déplacement rotatif du premier élément de comptage 410. Par contre, il n'y a pas ici d'élément intermédiaire rotatif pour interconnecter les premier et second éléments de comptage, mais c'est le premier élément de comptage qui comporte des moyens adaptés à coopérer avec le second élément de comptage.

Le compteur est disposé latéralement par rapport au corps du dispositif de distribution, et l'organe d'actionnement 430 transforme alors un déplacement axial du réservoir en un déplacement rotatif du premier élément de comptage 410. Dans ce cas, les deux éléments rotatifs 410, 420 du compteur tournent autour d'un axe de rotation 461 sensiblement perpendiculaire à ce déplacement axial. Avantageusement, le cycle d'actionnement du compteur peut démarrer au tout début de la course du réservoir, de sorte que le compteur est actionné avant que le produit ne soit distribué.

Le premier élément de comptage rotatif 410 forme la roue des unités et le second élément de comptage rotatif 420 forme la roue des dizaines, lesdits premier et second éléments de comptage coopérant pour définir ensemble et afficher dans une fenêtre de visualisation (non représentée) le nombre de doses de produit fluide distribué ou restant à distribuer dudit réservoir. De préférence, ce nombre est formé par une zone d'affichage dans laquelle le nombre est affiché horizontalement lorsque le dispositif de distribution est dans sa position d'utilisation normale, dans laquelle le corps est sensiblement vertical avec l'embout buccal disposé en bas. Ledit premier élément de comptage 410 coopère avec l'organe d'actionnement 430 qui est adapté à faire tourner ledit premier élément de comptage 410 à chaque actionnement dudit organe d'actionnement. Des moyens d'interconnexion sont adaptés à faire tourner ledit second élément de comptage 420 tous les dix actionnements dudit organe d'actionnement 430, et donc toutes les dix rotations dudit premier élément de comptage 410. Les deux éléments de comptage sont avantageusement assemblés de manière rotative autour du même axe de rotation 461.

Comme représenté sur les figures 10 à 15, le compteur comporte de préférence un corps de base 460, auquel peut être associé un couvercle (non représenté). Ledit corps de base forme l'axe de rotation 461 et comporte une ouverture 465 destinée à laisser passer l'élément d'actionnement 435. Le couvercle peut comporter une fenêtre de visualisation permettant à l'utilisateur de voir la zone d'affichage formée conjointement par lesdits premier et second éléments de comptage 410, 420. Le corps de base 460 peut comporter des moyens de fixation 467, tels que des pattes d'encliquetage, adaptés à fixer le bord externe du second élément de comptage 420, sans toutefois limiter sa capacité à tourner autour de son axe 461. Le second élément de comptage 420 retient le premier élément de comptage 410, qui lui-même retient l'organe d'actionnement 430 dans le corps de base 460. Ainsi, ledit compteur peut avantageusement être pré-assemblé pour former une unité de compteur, ladite unité de compteur pouvant comporter des moyens de fixation au corps du dispositif de distribution de produit fluide. De préférence, ces moyens de fixation sont formés sur ledit corps de base.

Comme représenté sur les figures 17a et 17b, ledit premier élément de comptage 410 peut comporter une première denture périphérique 411 coopérant à chaque actionnement avec une patte d'actionnement flexible 431 de l'organe d'actionnement 430. Ledit premier élément de comptage 410 est avantageusement sensiblement en forme de disque en étant pourvu d'une ouverture centrale traversante 415 adaptée à s'emmancher autour de son axe de rotation 461. La face supérieure dudit disque comporte une première partie de bord périphérique 412 recevant des indices de comptage 4120, tel qu'une ou plusieurs séries de chiffres de 0 à 9. L'exemple représenté sur la figure 10 montre deux séries de chiffres de 0 à 9, répartis sur ladite périphérie. La face inférieure dudit disque comporte ladite première denture périphérique 411, comme visible sur la figure 17b. De préférence, les dents de la première denture 411 sont orientées axialement. Comme visible sur la figure17a, la face supérieure dudit premier élément de comptage 410 peut comporter une partie plane centrale entourant l'ouverture centrale 415, prolongée radialement à l'extérieur par ladite première partie de bord périphérique 412 axialement surélevée par rapport à ladite partie centrale. La première partie de bord périphérique 412 se prolonge radialement à l'extérieur par une partie plane externe 413 qui ne s'étend pas sur toute la périphérie, mais qui est interrompue par au moins une, de préférence deux pattes flexibles 418, chacune comportant un ergot 419 à son extrémité. Les pattes flexibles 418 s'étendent de préférence de manière périphérique, et l'ergot 419 peut s'étendre perpendiculairement à sa patte respective 418, comme visible sur les figures 17a et 17b. Cette mise en œuvre permet de superposer le second élément de comptage 420 sur le premier élément de comptage 410 avec une épaisseur globale réduite.

Comme représenté sur les figures 16a et 16b, ledit second élément de comptage 420 peut comporter une seconde denture périphérique 421 adaptée à coopérer tous les dix actionnements de l'organe d'actionnement 430 avec un ergot 419 dudit premier élément de comptage 410. Avantageusement, ledit second élément de comptage est sensiblement en forme d'anneau, adapté à être disposé sur ladite partie externe 413 dudit premier élément de comptage 410. La face supérieure dudit anneau comporte une seconde partie de bord périphérique radialement externe 422 recevant des indices de comptage 4220, tel que les chiffres de 00 à 20, répartis sur ladite périphérie. Dans cet exemple, le compteur est donc capable de compter 200 doses. La face inférieure dudit anneau comporte ladite seconde denture périphérique 421, disposée radialement à l'intérieur, et s'étendant axialement vers le bas, et une troisième denture périphérique 426, disposée radialement à l'extérieur de ladite face inférieure. Cette troisième denture 426 est adaptée à coopérer avec au moins une projection prévue sur le corps de base 460 pour agir en tant que moyens anti-retour. Avantageusement, après assemblage des premier et second éléments de comptage 410, 420 autour de leur axe de rotation commun 461, ladite seconde partie de bord périphérique 422 dudit second élément de comptage 420 est disposée radialement à l'extérieur et sensiblement en contact de ladite première partie de bord périphérique 412 dudit premier élément de comptage 410, les surfaces supérieures desdites première et secondes parties de bord périphérique 412, 422 étant sensiblement alignées ou coplanaires pour former la zone d'affichage qui sera visible à travers la fenêtre de visualisation.

Le premier élément de comptage 410 comporte au moins un doigt déformable 418, de préférence deux doigts diamétralement opposés, le ou les doigt(s) déformable(s) étant adapté(s) à coopérer tous les dix actionnements avec une came solidaire du corps de base. Eventuellement, plusieurs cames peuvent être prévues. La seconde denture 421 du second élément de comptage 420 est destinée à coopérer avec l'ergot 419 d'un doigt déformable 418 du premier élément de comptage à chaque fois qu'il est déplacé vers sa position déformée par ladite came. Plus clairement, la came prévue dans ledit corps de base est adaptée à déformer élastiquement un doigt déformable 418 radialement vers l'intérieur, à chaque fois que l'ergot 419 dudit doigt déformable 418 coopère avec ladite came. Lorsque le doigt 418 n'est pas déformé, ledit ergot 419 ne coopère pas avec ladite seconde denture 421.

Dans l'exemple représenté sur les dessins, le premier élément de comptage 410 comporte deux doigts déformables 418 diamétralement opposés, ainsi que deux séries de chiffres 0 à 9 réparties sur la périphérie. Tous les dix actionnements, un des deux doigts déformables 418 coopère avec ladite came, de préférence prévue radialement à l'extérieur par rapport auxdits doigts, pour les déformer vers l'intérieur et ainsi permettre audit ergot 419 de coopérer avec la seconde denture 421 du second élément de comptage 420. Le second élément de comptage 420 est alors également entraîné en rotation. La troisième denture 426 du second élément de comptage 420 est destinée à coopérer avec des moyens anti-retour, par exemple une projection qui peut être solidaire du corps de base 460. Il est à noter que les moyens anti-retour pourraient également coopérer avec la seconde denture, auquel cas le second élément de comptage 420 pourrait ne comporter qu'une seule denture.

Un avantage du compteur de la présente invention est qu'il permet l'affichage en grande dimension sans augmenter l'encombrement du compteur. En particulier, l'exemple représenté permet pour un compteur de 200 doses, d'afficher des chiffres (unités d'une part, dizaines d'autre part) ayant une hauteur supérieure à 2 mm, de préférence environ 2,5 mm, et une largeur supérieure à 1,5 mm, de préférence environ 2 mm. Ceci représente une augmentation de la taille des chiffres allant jusqu'à 50% par rapport aux compteurs existants.

Dans l'exemple représenté, l'indicateur est adapté à indiquer le nombre de doses restant à distribuer de sorte que le chiffre affiché diminue à chaque actionnement. L'inverse est bien entendu également possible, à savoir un compteur qui compte le nombre de doses distribuées.

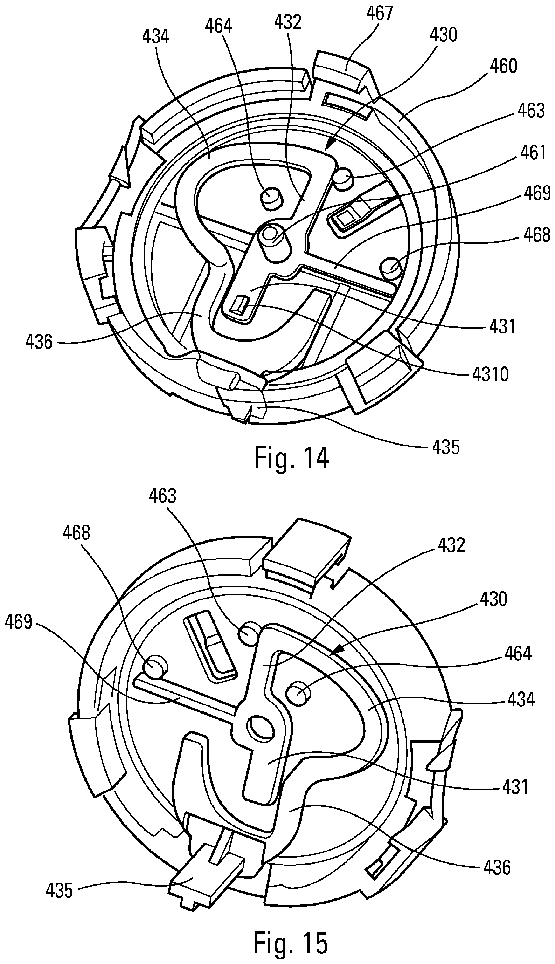

Avantageusement, ledit organe d'actionnement 430 est assemblé dans ledit corps de base 460 en étant emmanché autour de l'axe de rotation 461, comme visible notamment sur les figures 14 et 15. Cet organe d'actionnement comporte une patte 431 supportant un ergot 4310 adapté à coopérer avec la première denture 411 du premier élément de comptage 410. Cette patte 431 peut s'étendre vers le bas à partir de l'axe de rotation 461, lorsque le compteur est dans sa position verticale normale d'utilisation. Elle pourrait toutefois aussi s'étendre dans une autre direction, par exemple horizontalement dans la position d'utilisation. La patte 431 se prolonge d'un autre coté de l'axe de rotation 461 par une partie rectiligne sensiblement rigide 432, elle-même reliée à une partie élastiquement déformable 434 formant une boucle permettant de contourner l'axe de rotation 461. Cette partie élastiquement déformable 434 se prolonge axialement par une partie de support rigide 436 supportant l'élément d'actionnement 435, qui est déplaçable axialement. Avantageusement, des moyens de butée 463, 464 sont prévus pour former une butée au déplacement en rotation de la patte 431. Ces moyens de butée peuvent avantageusement être formés par deux projections 463, 464 du corps de base 460 qui peuvent coopérer avec la partie rectiligne sensiblement rigide 432 qui prolonge la patte 431, de l'autre coté de l'axe de rotation 461 dans l'exemple représenté sur les figures. D'autres moyens de butée sont aussi envisageables. Ainsi, à chaque actionnement, l'élément d'actionnement 435 est déplacé axialement vers le bas, provoquant la rotation de la patte 431 autour de l'axe de rotation 461. Lorsque la position de butée de la patte 431 est atteinte, l'élément d'actionnement 435 pourra continuer son déplacement axial grâce à la partie élastiquement déformable 434. Avantageusement, la patte 431 est sensiblement rigide axialement et est flexible dans une direction perpendiculaire au plan du corps de base 460. Ceci permet à la patte 431 de se déformer légèrement pour permettre à l'ergot 4310 de glisser sur la denture 411 et s'engrener dans la prochaine dent. Avantageusement, l'organe d'actionnement 430 comporte des moyens élastiques 469, tels qu'une lame élastique qui coopère avec un épaulement approprié 468 du corps de base 460 pour former ressort de rappel pour l'organe d'actionnement 430. Dans l'exemple représenté, cette lame élastique 469 s'étend perpendiculairement à la patte flexible 431. Bien entendu, si la patte flexible 431 s'étendait dans une autre direction (par exemple, celle de la lame élastique 469 sur la figure 14), alors cette lame élastique pourrait s'étendre aussi dans une autre direction. En particulier, la lame élastique 469 et la patte flexible 431 pourraient être inversées par rapport aux figures 14 et 15. La partie axialement déformable 434 de l'organe d'actionnement 430 supportant l'élément d'actionnement 435 permet de poursuivre le déplacement axial de l'élément d'actionnement 435 (et donc du réservoir) après que la position de butée de la patte 431 définie par la projection a été atteinte. Cette butée peut être formée de telle sorte qu'exactement une rotation d'une dent soit obtenue lorsque la position de butée est atteinte. L'actionnement de la valve nécessitant généralement une course supérieure, et donc un déplacement axial supérieur du réservoir, la partie déformable 434 de l'organe d'actionnement 430 permet la poursuite du déplacement axial du réservoir jusqu'à son terme. Par ailleurs, ce système permet d'actionner le compteur avant le début de la distribution du produit.

Le compteur du second mode de réalisation, de par ses composants et leur agencement, peut aussi être réalisé avec une faible épaisseur. En particulier, l'unité de compteur pré-assemblée, incluant le corps de base 460, l'organe d'actionnement 430, et les premier et second éléments de comptage 410, 420 peut avoir une épaisseur inférieure ou égale à 7 mm, avantageusement inférieure à 6 mm, notamment inférieure à 5 mm. Ces mesures ne tiennent bien entendu pas compte de l'élément d'actionnement 435 qui se projette hors de l'unité de compteur pré-assemblée dans le sens de l'épaisseur.

## Revendications

1. Dispositif de distribution de produit fluide ou pulvérulent (200) comportant un réservoir, un organe de distribution, tel qu'une valve doseuse, monté sur ledit réservoir, et un corps (201) incorporant un orifice de distribution (203), ledit réservoir étant déplaçable dans ledit corps (201) pour distribuer du produit fluide ou pulvérulent, ledit dispositif de distribution (200) comportant un compteur de doses pour compter le nombre de doses de produit fluide ou pulvérulent distribuées ou restant à distribuer à partir dudit dispositif de distribution de produit fluide (200), ledit compteur comportant un premier élément de comptage rotatif (110 ; 410) formant la roue des unités et un second élément de comptage rotatif (120 ; 420) formant la roue des dizaines, lesdits premier et second éléments de comptage (110, 120 ; 410, 420) coopérant pour définir ensemble et afficher dans une fenêtre de visualisation (151) ledit nombre de doses, ledit premier élément de comptage (110 ; 410) coopérant avec un organe d'actionnement (130 ; 430) adapté à faire tourner ledit premier élément de comptage (110 ; 410) à chaque actionnement dudit organe d'actionnement (130 ; 430), le compteur comportant un corps de base (160 ; 460), incorporant l'axe de rotation (161 ; 461) desdits premier et second éléments de comptage, et une ouverture (165 ; 465), ledit compteur pouvant être pré-assemblé dans ledit corps de base (160 ; 460) pour former une unité de compteur pré-assemblée, ledit organe d'actionnement (130 ; 430) comportant un élément d'actionnement (135 ; 435) faisant saillie hors dudit corps de base (160 ; 460) à travers ladite ouverture (165 ; 465), ladite unité de compteur comportant des moyens de fixation audit corps (201) dudit dispositif de distribution de produit fluide (200),lesdits moyens de fixation étant formés sur ledit corps de base, ledit compteur (100) étant fixé latéralement au corps (201), l'actionnement dudit dispositif étant réalisé par une pression manuelle axiale de l'utilisateur sur le réservoir et l'actionnement dudit compteur étant réalisé par ledit déplacement axial dudit réservoir qui coopère avec ledit élément d'actionnement (135 ; 435) de l'organe d'actionnement (130 ; 430).

2. Dispositif selon la revendication 1, dans lequel ledit corps de base est associé à un couvercle adapté à être fixé sur ledit corps de base, après assemblage du compteur dans le corps de base.

3. Dispositif selon la revendication 1 ou 2, dans lequel l'unité de compteur pré-assemblée, incluant le corps de base (160), l'organe d'actionnement (130), l'élément intermédiaire (140), les premier et second éléments de comptage (110, 120) et le couvercle (150) présente une épaisseur inférieure ou égale à 7 mm, avantageusement inférieure à 6 mm, notamment inférieure à 5 mm.

4. Dispositif selon la revendication 1 ou 2, dans lequel l'unité de compteur pré-assemblée, incluant le corps de base (460), l'organe d'actionnement (430), et les premier et second éléments de comptage (410, 420) présente une épaisseur inférieure ou égale à 7 mm, avantageusement inférieure à 6 mm, notamment inférieure à 5 mm.

## Patentansprüche

1. Abgabevorrichtung für ein Fluid- oder Pulverprodukt (200) aufweisend einen Behälter, ein Abgabeelement wie beispielsweise ein Dosierventil, das auf dem Behälter befestigt ist, und einen Körper (201) mit einer Abgabeöffnung (203), wobei der Behälter in dem Körper (201) bewegbar ist, um Fluid- oder Pulverprodukt abzugeben, die Abgabevorrichtung (200) aufweisend einen Dosiszähler, um die Anzahl von Fluid- oder Pulverproduktdosen zu zählen, die aus der Fluidprodukt-Abgabevorrichtung (200) abgegeben wurden oder noch abzugeben sind, der Zähler aufweisend ein erstes Drehzählelement (110; 410), das das Rad der Einer bildet, und ein zweites Drehzählelement (120; 420), das das Rad der Zehner bildet, wobei das erste und zweite Zählelement (110, 120; 410, 420) zusammenwirken, um zusammen die Anzahl von Dosen zu definieren und in einem Visualisierungsfenster (151) anzuzeigen, wobei das erste Zählelement (110; 410) mit einem Betätigungsglied (130; 430) zusammenwirkt, das geeignet ist, das erste Zählelement (110; 410) bei jeder Betätigung des Betätigungsglieds (130; 430) zu drehen, der Zähler aufweisend einen Grundkörper (160; 460), der die Drehachse (161; 461) des ersten und zweiten Zählelements enthält, und eine Öffnung (165; 465), wobei der Zähler in dem Grundkörper (160; 460) vormontiert sein kann, um eine vormontierte Zählereinheit zu bilden, das Betätigungsglied (130; 430) aufweisend ein Betätigungselement (135; 435), das durch die Öffnung (165; 465) aus dem Grundkörper (160; 460) herausragt, die Zählereinheit aufweisend Mittel zur Befestigung am Körper (201) der Fluidprodukt-Abgabevorrichtung (200), wobei die Befestigungsmittel auf dem Grundkörper ausgebildet sind, wobei der Zähler (100) seitlich am Körper (201) befestigt ist, wobei die Betätigung der Vorrichtung durch ein axiales manuelles Drücken des Benutzers auf den Behälter und die Betätigung des Zählers durch die axiale Bewegung des Behälters, der mit dem Betätigungselement (135; 435) des Betätigungsglieds (130; 430) zusammenwirkt, durchgeführt wird.

2. Vorrichtung nach Anspruch 1, wobei der Grundkörper mit einem Deckel in Verbindung steht, der geeignet ist, nach Montage des Zählers in den Grundkörper auf dem Grundkörper befestigt zu sein.

3. Vorrichtung nach Anspruch 1 oder 2, wobei die vormontierte Zählereinheit, die den Grundkörper (160), das Betätigungsglied (130), das Zwischenelement (140), das erste und zweite Zählelement (110, 120) und den Deckel (150) enthält, eine Dicke kleiner oder gleich 7 mm, vorteilhafterweise kleiner als 6 mm, insbesondere kleiner als 5 mm aufweist.

4. Vorrichtung nach Anspruch 1 oder 2, wobei die vormontierte Zählereinheit, die den Grundkörper (460), das Betätigungsglied (430), das erste und zweite Zählelement (410, 420) enthält, eine Dicke kleiner oder gleich 7 mm, vorteilhafterweise kleiner als 6 mm, insbesondere kleiner als 5 mm aufweist.

## Claims

1. A fluid or powder dispenser device (200) comprising a reservoir, a dispenser member, such as a metering valve, that is mounted on said reservoir, and a body (201) incorporating a dispenser orifice (203), said reservoir being movable in said body (201) so as to dispense the fluid or powder, said dispenser device (200) including a dose counter for counting the number of doses of fluid or powder that have been dispensed or that remain to be dispensed from a fluid dispenser device (200), said counter including a first rotary counter element (110; 410) forming a units wheel, and a second rotary counter element (120; 420) forming a tens wheel said first and second counter elements (110, 120; 410, 420) co-operating with each other to define and to display in a viewing window (151), said number of doses, said first counter element (110; 410) co-operating with an actuator member (130; 430) that is adapted to cause said first counter element (110; 410) to turn each time said actuator member (130; 430) is actuated,the counter including a base body (160; 460), that incorporates a pivot pin (161; 461) for said first and second counter elements, and an opening (165; 465), said counter possibly being pre-assembled in said base body (160; 460) so as to form a pre-assembled counter unit, said actuator member (130; 430) including an actuor element (135; 435) projecting out of said base body (160; 460) through said opening (165; 465), said counter unit including fastener means for fastening to a body (201) of a fluid dispenser device (200), said fastener means are formed on said base body, said counter (100) being fastened to the body (201) laterally, said device being actuated by the user pressing axially on the reservoir and said counter being actuated by said axial movement of said reservoir that co-operates with said actuator element (135; 435) of the actuator member (130; 430).

2. Device according to claim 1, wherein said base body is associated with a lid that is adapted to be fastened on said base body, after the counter has been assembled in the base body.

3. Device according to claim 1 or 2, wherein the pre-assembled counter unit, comprising the base body (160), the actuator member (130), the intermediate element (140), the first and second counter elements (110, 120) and the lid (150) presents thickness that is less than or equal to 7 mm, advantageously less than 6 mm, in particular less than 5 mm.

4. Device according to any one of claims 1 or 2, wherein the pre-assembled counter unit, comprising the base body (460), the actuator member (430), and the first and second counter elements (410, 420) presents thickness that is less than or equal to 7 mm, advantageously less than 6 mm, in particular less than 5 mm.
